# EUROPEAN PATENT APPLICATION

(11) **EP 1 980 197 A1**
(43) Date of publication of application: **15.10.2008**
(21) Application number: 08154095.7
(22) Date of filing: 04.04.2008
(51) Int. Cl.: A61B 3/028, A61B 3/032

(54) **Lens Selection System**

(30) Priority: 10.04.2007 GB 0706719
(71) Applicant: Elhami Limited, East Kilbride G74 4NB (GB)
(72) Inventor: Moshirya, Ali-Reza, Hamilton, Strathclyde ML3 7EX (GB); Nelson, William Greer, Glasgow, Strathclyde G61 4DS (GB)
(74) Representative: Brown, Deborah Frances

(57) **Abstract**

A lens selection system is described. The lens system comprises a housing; at least one lens arranged to allow a view into the housing by a user; a support mechanism on which the least one lens is mounted, the support mechanism having a part that is fixed relative to the housing; and a light source located within the housing.

## Description

The present invention relates to a lens selection system. The invention relates especially, but not exclusively to self-selecting lenses for reading glasses, e.g. to correct a sight deficit, for example presbyopia/long-sightedness.

When a person recognises they have a sight problem, they may go to an opticians, have their sight professionally tested, and have glasses especially made for their eyesight. Alternatively, the person can go to a shop and select one of a pair of ready-made glasses themselves. This might be cheaper than visiting the opticians and, if the optical difficulty is a common one, the range of ready-made glasses will probably include lenses that are a good match for that person. In this case, the person may test his vision by looking, through a variety of different lenses, at a reading chart (which typically has rows of letters of different sizes). The person can then find out which lens strength is most helpful to correct his vision. After having identified the strength of lens that he requires, the person can then choose a pair of ready-made glasses that has this particular lens strength.

These tests are carried out under the available ambient light. The level of light in different rooms can vary greatly, as can the level of light in different places in the same room. A problem can arise when these tests are conducted under insufficient light, which can adversely affect the result, without the person becoming aware of this. The person may then select and purchase glasses which are too strong or too weak for him. If the test had been conducted in another shop/location/time of day, the person might have selected a different strength pair of glasses.

The person is unlikely to realise he has purchased a less than ideal pair of glasses, and will most probably trust the test result. He is highly unlikely to shop around, to see whether he chooses the same strength glasses in each shop.

A system of self-selecting a lens from a plurality of lenses located on a dial is known. The dial is rotatable to present different strength lenses to a person's eye and the person selects the lens that he thinks helps him to see the best. The person can then choose a pair of ready-made glasses that have this lens strength.

A problem can arise when the dial is not rotated to exactly the right position. For example, a lens of strength 1.25 dioptres has that precise strength only at the exact centre of this lens. If the lens is not accurately positioned, the user may be inadvertently looking through another part of the lens, which is not exactly 1.25 dioptres in strength. The person is unlikely to realise this, or to be able to exactly line up the dial with his eye, so he may select and purchase a pair of glasses that are less than ideal for his sight.

According to a first aspect of the present invention there is provided a lens selection system, comprising:
a housing;
at least one lens arranged to allow a view into the housing by a user;
a support mechanism on which the least one lens is mounted, the support mechanism having a part that is fixed relative to the housing; and
a light source located within the housing.

A reading chart can be located in the housing at a particular distance from the lens, so the distance between the lens and the reading chart can be controlled. This ensures that users do not inadvertently test different lenses at different distances from the reading chart, thereby arriving at a potentially erroneous result. Locating a light source within the housing means that the level of light under which sight tests are conducted can be controlled, to obtain consistent results at any time and location.

Preferably, the light source comprises a light emitting diode.

Light emitting diodes have the advantage that they are long-lasting and efficient.

Optionally, the light source is provided with a diffuser. The diffuser can be used to spread the light evenly.

Preferably, a plurality of lenses are mounted on the support mechanism, and the support mechanism has a part that is moveable with respect to the housing to present a selected lens to the user.

Optionally, the moveable part of the support mechanism comprises at least one rotary dial.

Optionally, the moveable part of the support mechanism comprises two rotary dials that are coupled together such that rotation of one dial will also rotate the other dial.

Typically, the rotary dials are coupled together by interlocking cog teeth provided on the dials.

Alternatively, the moveable part of the support mechanism comprises an elongate conveyor or a continuous track conveyor.

Alternatively, any means of presenting a lens to a user can be used. Hence, it is not essential for the moveable part of the support mechanism to comprise one or more rotary dials.

Preferably, the moveable part of the support mechanism is moveable into a number of discrete positions.

Optionally, the system includes a locating means that resists movement of the moveable part of the support mechanism away from the discrete positions.

The locating means can therefore effectively define the discrete positions. The locating means can typically be arranged such that it locates the selected lens in its most ideal position, e.g. when the centre of the lens is exactly aligned with the visual path to the reading chart. This can help ensure that the user will always be looking through the exact centre of the lens. This is important, since the lens strength a few millimetres away from the lens centre is typically not the same as the lens strength at the centre of the lens.

Typically, the locating means comprises a catch and a series of grooves, the catch being selectively engageable with the grooves.

Optionally, the catch is mounted on the fixed part of the support mechanism and the grooves are located on the moveable part of the support mechanism.

Optionally, the catch comprises a ball catch.

Typically, the ball catch comprises a ball bearing that is located in a recess in the fixed part of the support mechanism, and the ball is spring-urged such that it tends to protrude from the recess and into contact with the moveable part of the support mechanism.

This helps ensure that the catch will automatically be urged into a groove, whenever such a groove is located adjacent thereto.

When the ball bearing is located in a groove, the force of the spring is typically sufficient to hold the moveable part of the support mechanism fixed against unintentional movement, but the spring force can be overcome by intentional movement of the moveable part of the support mechanism.

Preferably, the system includes a light control means for controlling the level of light inside the housing.

Controlling the level of light helps to ensure that consistent results are obtained wherever and whenever a sight test is conducted.

Typically, the light control means comprises electronic circuitry.

Typically, the electronic circuitry is provided with a power supply and the electronic circuitry also includes means to ensure the light source will be activated only when a threshold voltage is provided by the power supply.

This is useful to prevent a sight test from being conducted under an insufficient level of light.

Optionally, the electronic circuitry consists of non-intelligent logic components.

Alternatively, the electronic circuitry comprises a Central Processing Unit.

Preferably, the system has a powered up mode and a stand-by mode.

This helps to prolong the life of the power supply (e.g. battery).

Optionally, the powered up mode is activated by movement of the moveable part of the support mechanism.

Optionally, the electronic circuitry includes a timer that is adapted to activate the stand-by mode if the moveable part of the support mechanism has been stationary for a certain period of time.

Preferably, the light control means prevents power being supplied to the light source when the system is in the stand-by mode.

Preferably, the electronic circuitry includes a detector for detecting the position of the moveable part of the support mechanism.

Optionally, the detector comprises at least one switch, adapted to detect the presence or absence of an adjacent magnet on the moveable part of the support mechanism.

Alternatively, the detector comprises at least one optical sensor.

The optical sensor may be adapted to detect the presence or absence of an adjacent protrusion/hole/other feature on the moveable part of the support mechanism.

Preferably, the electronic circuitry includes a display for presenting information to a user.

Typically, the display comprises a digital or LED display.

Typically, the information to be presented on the display comprises the lens strength of the lens presented to the user, and the electronic circuitry includes means to convert the detected position of the support mechanism into the corresponding lens strength.

Preferably, the electronic circuitry is adapted to display information relating to the lens strength only when the support mechanism is in one of the discrete positions.

Such embodiments may be advantageously arranged such that the user is only given information on the lens strength when the selected lens is in its most ideal position for making the sight test, e.g. when the lens is exactly aligned with the visual path to the reading chart.

Thus, if a lens is not exactly in the required position, the user is not given the information about the lens strength. The user would therefore become aware that the lenses are misaligned, if no information, or an error message, is displayed to him on the display.

Typically, a reading chart is located inside the housing.

Optionally, at least one mirror is located in the visual path between the lens and the reading chart.

The length of the visual path from the lens to the reading chart can be diverted using one or more mirrors in order to provide a more compact system.

The incorporation of the mirror can ensure that the depth of the housing can be compact. This can be advantageous for incorporating the system as part of a shop display e.g. an upper part of a glasses presentation stand, which may not have a large horizontal depth. As compared to embodiments without a mirror, the mirror effectively allows for an increase in the length of the visual path (focal distance) from the lens to the reading chart without increasing the depth of the housing. This is achieved by making use of spare vertical space between the top of the presentation stand and the ceiling. In some embodiments, the mirror diverts the visual path through 90 degrees.

Preferably, the length of the visual path between the lens and the reading chart is adjustable.

Hence, the lens selection system can be used for sight tests over a number of different distances, which is useful because books, laptop screens and desktop computer screens are all typically read from different distances.

According to a second aspect of the present invention there is provided a lens selection system, comprising:
a housing;
a plurality of lenses; and
a support mechanism on which the lenses are mounted, the support mechanism having a part that is fixed relative to the housing, and another part that is moveable to present a selected lens to the user;
wherein the moveable part of the support mechanism is selectively moveable into a number of discrete positions, at least some of which present a respective lens to the user; and
wherein the system includes a locating means that resists movement of the moveable part of the support mechanism away from the discrete positions.

Optionally, the second aspect of the invention includes a light source located within the housing. Optionally, the second aspect of the invention includes any or all of the optional features of the first aspect of the invention.

The locating means can therefore effectively define the discrete positions. The locating means can typically be arranged such that it locates the selected lens in its most ideal position, e.g. when the lens is exactly aligned with the visual path to the reading chart. This can ensure that the user will always be looking through the exact centre of the lens; hence, more accurate test results can be obtained.

According to a third aspect of the present invention there is provided a lens selection system, comprising:
a housing;
a plurality of lenses;
a support mechanism on which the lenses are mounted, the support mechanism having a part that is fixed relative to the housing, and another part that is moveable into a number of discrete positions, at least some of which present a respective lens to the user; and
wherein the system includes electronic circuitry that is adapted to detect the position of the moveable part of the support mechanism and to present information relating to the selected lens to the user.

Optionally, the third aspect of the invention includes a light source located within the housing. Optionally, the third aspect of the invention includes any or all of the optional features of the first aspect of the invention.

Such embodiments may be advantageously arranged such that the user is only given information on the lens strength when the selected lens is in its most ideal position for making the sight test, e.g. when the lens is exactly aligned with the visual path to the reading chart.

Thus, if a lens is not exactly in the required position, the user is not given the information about the lens strength. The user would therefore become aware that he has misaligned the presenting means, if no information, or an error message, is displayed to him on the display.

An embodiment of the invention will now be described, by way of example only, and with reference to the following drawings, in which:-
Fig 1 shows a side elevation of a lens selection system according to the invention;
Fig 2 shows a side elevation of a lower part of the lens selection system of Fig 1;
Fig 3 shows a front view of two dials of a support mechanism of Fig 1;
Fig 4 shows a front view of the dials of Fig 3 mounted on a back plate;
Fig 5 shows a side elevation of part of the lens selection system of Fig 1, showing details of the support mechanism;
Fig 6 shows a front view of a circuit board of the lens selection system;
Fig 7 shows a rear view of the circuit board of Fig 6; and
Fig 8 shows a flow chart of the inputs and outputs of the lens selection system of Fig 1.

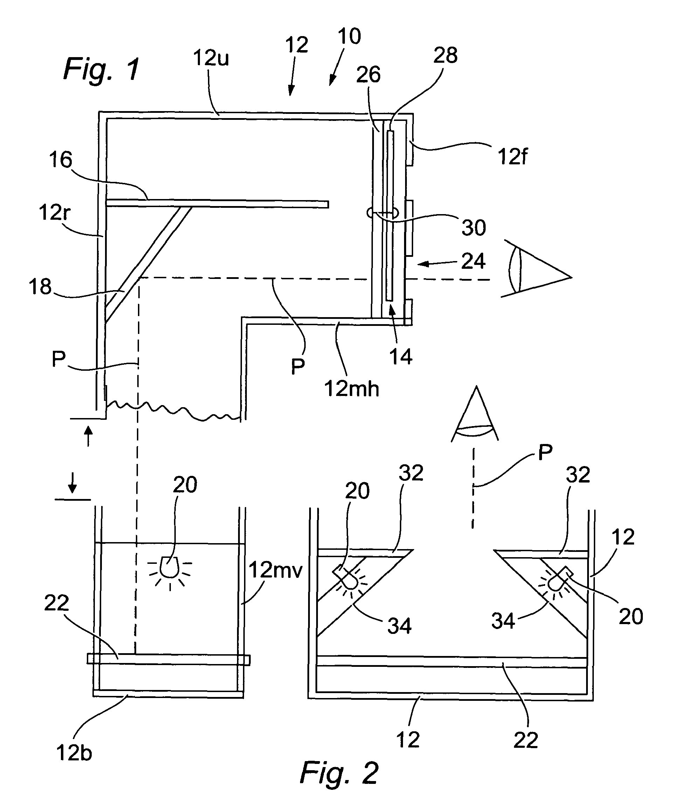

Referring now to Fig 1, a lens selection system 10 comprises an L-shaped housing 12, in which is located a support mechanism 14, a partition strut 16, a mirror 18, a light source comprising two light emitting diodes 20, and a reading chart 22.

The housing 12 has a front wall 12f, a rear wall 12r, an upper wall 12u, and a base wall 12b. The L-shape is completed by a minor vertical wall 12mv parallel to the rear wall 12r, and a minor horizontal wall 12mh parallel to the upper wall 12u.

A viewing slot 24 is located in the front wall 12f.

The support mechanism 14 comprises a back plate 26, two dials 28, and two mounting pivots 30, which mount the dials 28 on the back plate 26. The back plate 26 is fixed to the housing 12, behind the front wall 12f of the housing 12. The dials 28 are rotatably mounted on the back plate 26 by the pivots 30; hence the dials 28 are rotatable relative to the housing 12.

A plurality of lenses of different optical strengths are mounted on each dial 28, as will be explained in more detail below. Rotation of the dials 28 causes the plurality of lenses on the dials 28 to pass behind the viewing slot 24. Thus, a user can look into the housing 12 through the viewing slot 24 and the lenses, along a visual path P.

The partition strut 16 is fixed to the housing 12 and provides a support for an upper edge of the mirror 18. A lower edge of the mirror 18 is fixed to a rear wall 12r of the housing 12. The mirror is inclined at 45 degrees to the rear wall 12r, such that light rays hitting the mirror 18 at 45 degrees will be reflected through 90 degrees. The visual path P representing the passage of a light ray between the reading chart 22 and the user's eye is also reflected through 90 degrees.

The reading chart 22 is mounted slightly in front of the base wall 12b and parallel to the base wall 12b. However, the reading chart 22 need not be located near the base wall 12b, and may alternatively be moved closer to the mirror 18. Hence, the position of the reading chart 22 is adjustable to vary the length of the visual path P (focal distance) between the lens and the reading chart 22. Hence, the lens selection system 10 can be used to test sight over a number of different distances, which is useful because books, laptop screens and desktop computer screens are all typically read from different distances.

The light source is shown schematically in Fig 1, and the two light emitting diodes 20 which comprise the light source are shown in Fig 2. Partition struts 32 extend inwards into the housing 12, parallel to the reading chart 22 to form a part-rectangular shape at the bottom of the housing 12. A gap between the partition struts 32 allows the visual path P to pass between the struts 32. Thus, a user looking into the housing 12 can see the reading chart 22 using the mirror 18.

One light emitting diode 20 is mounted underneath each of the partition struts 32, angled inwardly and towards the reading chart 22. In front of each light emitting diode 20 is a diffuser 34, which spreads out light.

The incorporation of the mirror 18 can ensure that the depth of the housing 12 (the distance from the front wall 12f to the rear wall 12r) can be compact. This can be advantageous for incorporating the system 10 as part of a shop display e.g. an upper part of a glasses presentation stand, which may not have a large horizontal depth. As compared to embodiments without a mirror, the mirror 18 effectively allows for an increase in the length of the visual path P (focal distance) from the lens to the reading chart 22 without increasing the depth of the housing 12. This is achieved by making user of spare vertical space between the top of the presentation stand and the ceiling.

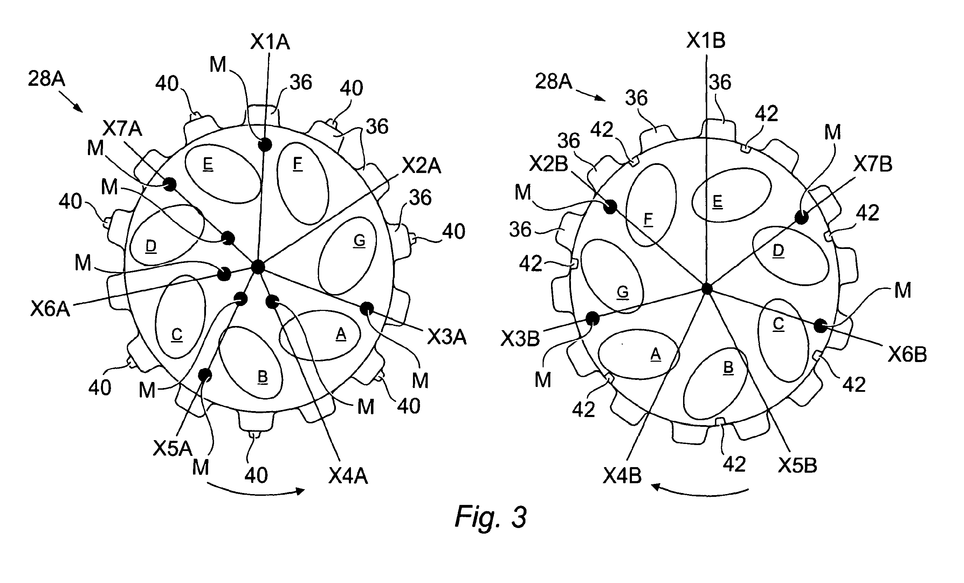

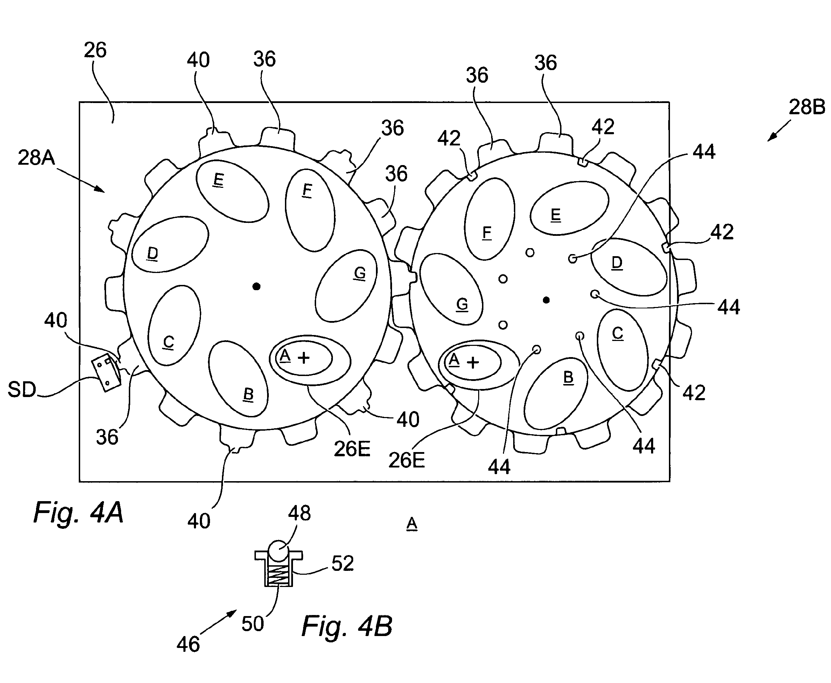

The support mechanism 14 is shown in more detail in Figs 3 to 5. The two dials 28A, 28B comprise rotary dials, each of which is provided with 14 interlocking cog teeth 36. When mounted on the back plate 26 (see Fig 4), the cog teeth 36 intermesh to couple the dials 28A, 28B together such that rotation of one dial will also rotate the other dial.

Seven lenses A to G are mounted on each dial 28A, 28B in a circular formation. Different pairs of lenses A-G have different lens strengths, but the lenses in each pair have the same strength as each other. The lenses A-G are preferably optical lenses, as opposed to injection-moulded plastic lenses. This contributes to a more accurate test.

In Figs 3 and 4, the rotational position of the dials 28A, 28B is such that the longitudinal axes of the pair of lenses A are parallel and the lenses A are presented to the viewing slot 24 in the housing 12. This position will hereinafter be known as the alignment configuration of lenses A. The dials 28A, 28B are dimensioned such that in Fig 4, the optical centres of the lenses A (marked with crosses) are around 62mm (an average eye width) apart.

Each pair of lenses B-G can be brought into their own alignment configuration (corresponding to the present position of lenses A) by rotating the dials 28A, 28B.

Hence, the dials have seven alignment configurations (discrete positions), corresponding to the alignment of each of the pairs of lenses A-G.

Each of these alignment configurations corresponds to two vertical alignment axes, one on each dial. These alignment axes are designated X1A-X7A on dial 28A and X1B-X7B on dial 28B. For example, the vertical alignment axis X1A is a line vertically upwards from the centre of the dial 28A when the lenses A are in their alignment configuration. In an alternative embodiment, the vertical alignment axes may not pass through the centres of the dials 28A, 28B, and may be offset relative to the centres of the dials.

Located along each alignment axis are either 0, 1 or 2 indicator elements, whose presence or absence can be detected. The indicator elements may be magnets, holes or raised segments. In this example, they are magnets M.

On the dial 28A, some of the magnets M are provided at a first radial distance along the alignment axes X1A-X7A, and some are provided at a second radial distance; these will be termed "outer" and "inner" magnets M. The inner magnets M are provided near the centre of the dial 28A, whereas the outer magnets M are provided near the circumference of the dial 28A. On the dial 28B, magnets M are provided near the circumference only, and there are no "inner" magnets.

The purpose of the magnets M will be explained later.

Referring particularly to Fig 4A, in addition to the dials 28A, 28B, Fig 4A also shows the back plate 26. The back plate 26 includes eye holes 26E which are larger than, but approximately correspond in shape to the aligned lenses A. The dial 28B extends past the right hand end of the back plate 26, allowing the dial 28B to be manually turned by hand (an aperture in the housing 12 is provided for such manual access). As explained above, rotation of the dial 28B will automatically also rotate the dial 28A, via the interlocking cog teeth 36.

Fig 4A also shows an additional switch SD, raised areas 40 on every second cog tooth 36 on the dial 28A, and grooves 42 located between every second cog tooth 36 on the dial 28B. The raised areas 40 engage in the grooves 42 when the dials 28A, 28B are rotated.

Switch SD is located at the bottom left of the dial 28A, at a location such that the raised areas 40 will contact the switch SD when they are aligned therewith, but the cog teeth 36 without raised areas will not contact the switch SD.

Since there are fourteen cog teeth 36, seven of which have raised areas 40, there is one raised area 40 per lens. The switch SD is positioned so that, in each of the alignment configurations, one of the raised areas 40 is in contact with the switch SD. For example, as shown in Fig 4, the lenses A are in their alignment configuration and the switch SD contacts one of the raised areas 40. If the dials 28A, 28B are rotated to align the lenses B, then another raised area 40 will contact the switch SD in this position, and so on.

Also shown in Fig 4A are seven, equally spaced, arcuate grooves 44 that are located in a circular path on the dial 28B.

Fig 4B shows a ball catch 46, for engagement with these grooves 44. The ball catch 46 and the grooves 44 together form a locating means that resists movement of the dial 28B away from the alignment configurations, as will now be explained.

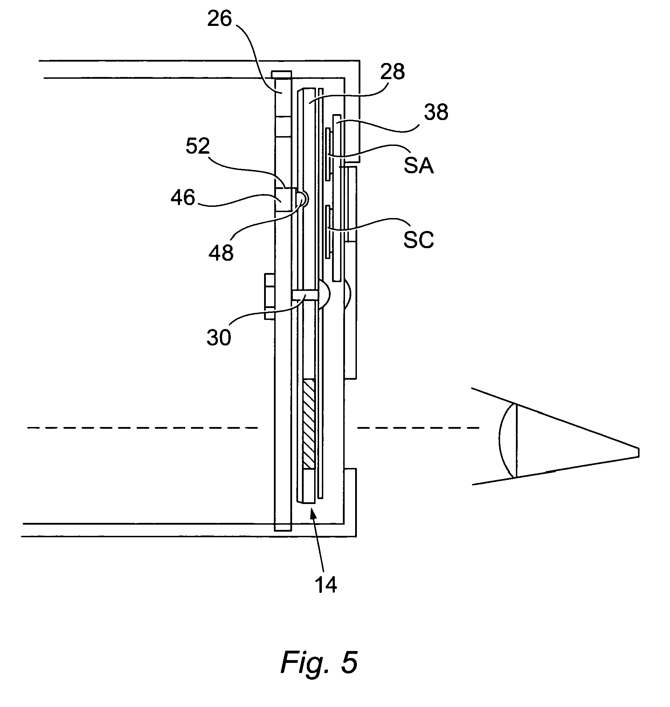

The ball catch 46 comprises a ball bearing 48, a spring 50 and a retainer plate (not shown). The ball catch 46 is located in a cylindrical recess 52 in the back plate 26 (see Fig 5). The spring 50 loads the ball bearing 48 such that it is urged out of the recess 52.

The ball catch 46 and arcuate grooves 44 are located such that the ball catch 46 engages one of the arcuate grooves 44 when a respective pair of lenses are exactly aligned in the required alignment configuration.

For example, when the lenses A are exactly aligned, the ball catch 46 engages with the uppermost groove 44 of Fig 4A, by the spring 50 pushing the ball bearing 48 into that arcuate groove 44. The ball catch 46 now resists further rotational movement of the dials 28A, 28B, which helps to keep the dials 28A, 28B in an exactly aligned position, against any (possibly accidental) movement away from these positions. A user taking a sight test feels this resistance and is reassured that the lenses A are exactly aligned in the correct position for his test. The resistance of the spring 50 is easily overcome if the dials 28A, 28B are intentionally rotated.

Using the ball catch 46 and grooves 44 is particularly beneficial because lens strength is typically highly dependent on whether or not the user is looking through the exact centre of the lens. Even a point a few millimetres away from the centre (which the user would probably not identify as misaligned) can affect the result. The ball catch 46 and grooves 44 ensure that the alignment is exact.

Hence, for each of the seven alignment configurations of the dials 28A, 28B, the ball catch 46 is engaged with one of the arcuate grooves 44 and the switch SD is in contact with a raised area 40 of a cog tooth 36 of the dial 28A.

At each position intermediate to these seven alignment configurations, the ball catch 46 is not engaged, and the switch SD is not contacted. Thus, the engagement of the ball catch 46 effectively defines the alignment configurations.

Hence, the moveable part of the support mechanism (the dials 28A, 28B) is moveable into a number of discrete positions (the seven alignment configurations). The system 10 includes a locating means (ball catch 46 and grooves 44) that resists movement of the dials 28A, 28B away from the discrete positions.

Referring to Fig 5, the relative alignment of the components of Figs 4A and 4B is shown. Also shown in Fig 5 are two lens securing plates 50 which are located on each side of the dials 28A, 28B, and which secure the lenses A-G in their respective dial 28A, 288.

Figs 5, 6 and 7 show a circuit board/switch mounting plate 38. The circuit board 38 is mounted to the back plate 26 at a location above the pivots 30, and adjacent to the upper parts of the dials 28A, 28B.

The circuit board 38 includes a light control means, which comprises electronic circuitry (not shown) that is connected to the light emitting diodes 20 via a separate cable assembly. The cable assembly includes a power regulator which is set at +8V DC. This provides a stable supply to drive the light emitting diodes 20. The current can be adjusted by changes to the regulator and resistance within the circuit to ensure a suitable light level is maintained. The light emitting diodes 20 are connected in series and their current is set at 22mA.

The electronic circuitry is provided with its own power supply, which comprises a battery of +12V DC. The electronic circuitry controls a flow of electricity from the battery 60 to the light emitting diodes 20. Therefore, the electronic circuitry controls the level of light inside the housing 12.

Controlling the level of light helps to ensure that consistent results are obtained, no matter where the test is conducted.

The electronic circuitry includes means to ensure the light emitting diodes 20 are activated only when a threshold voltage is provided by the power supply. This is useful to prevent the test from being conducted under an insufficient level of light. Such means is a battery voltage measuring circuit, which is described below.

In this example, the electronic circuitry consists of non-intelligent logic components.

The electronic circuitry also includes a detector for detecting the position of the dials 28A, 28B and means to convert the detected position of the dials into a corresponding lens strength, as will now be described.

The detector for detecting the position of the dials 28A, 28B includes three microswitches SA, SB and SC (reed switches), which are provided on the circuit board 38.

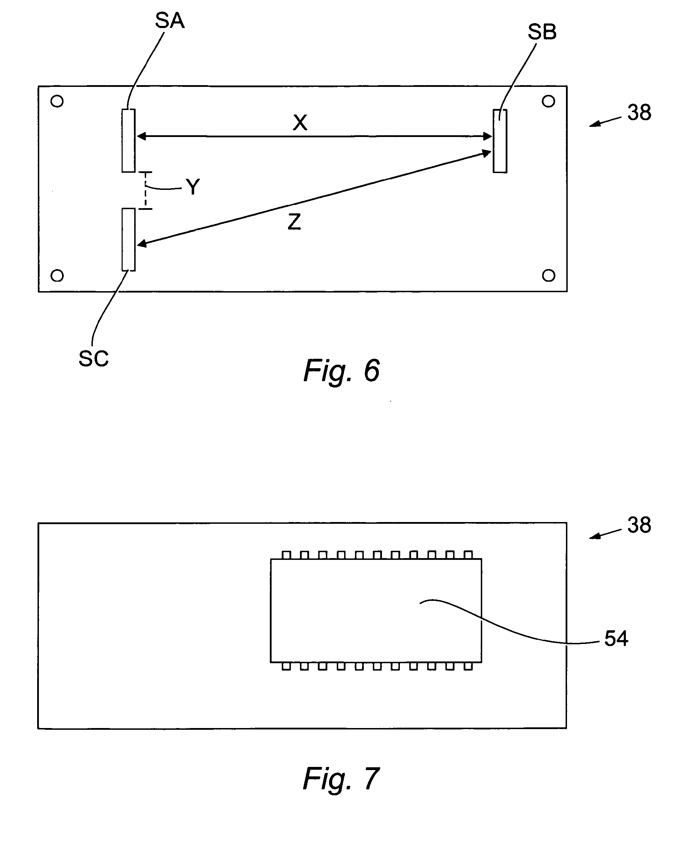

Fig 6 shows the side of the circuit board 38 that faces the dials 28A, 28B, and on which the switches SA, SB and SC are mounted. The distance X between switches SA and SB is equal to the horizontal distance between the centres of the dials 28A, 28B. The distance Y between switches SA and SC is equal to the distance between the outer and inner magnets M of the dial 28A.

Fig 7 shows the other side of this circuit board 38, on which is mounted a display 54 which comprises a 3.5 digit LCD display module for presenting information to a user. The information to be presented on the display 54 is intended to be principally the lens strength of the lens presented to the user, although other/alternative information may also be displayed.

The switch SA is located parallel to the vertical axis of symmetry of dial 28A, at a radial position corresponding to the outer magnets M on the dial 28A. The switch SC is located directly below the switch SA, at a radial position corresponding to the inner magnets M. The switch SB is located on the vertical axis of symmetry of the dial 28B, at a radial position corresponding to the outer magnets M on the dial 28B.

The magnets M are arranged so that each alignment configuration of the dials 28A, 28B corresponds to a unique combination of inner and outer magnets on the dials 28A, 28B. For example, referring to Fig 3, when the lenses A are aligned (as shown), axes X1 A and X1 B are vertical. Therefore, switch SA will detect an outer magnet M on the dial 28A, whilst switches SB and SC do not detect any magnets.

If the dials 28A, 28B are rotated so that lenses B are aligned, the axes X2A and X2B are now vertical. In this position, neither of switches SA or SC will detect a magnet, but switch SB will detect a magnet.

If the dials are rotated so that lenses C are aligned, the axes X3A, X3B are vertical. Switches SA and SB will detect magnets, but not switch SC, and so on, for the lenses D, E, F and G.

The microswitches SA, SB and SC are sensitive to magnetism, so that they detect the presence of an adjacent magnet M, and they respond by producing an electrical signal. The electronic circuitry interprets the activation of the switches according to the following rules. If switch SA is activated, a signal of value 1 is produced. If switch SB is activated, a signal of value 2 is produced. If switch SC is activated, a signal of value 4 is produced. These values are then summed to produce an overall value.

The following table shows the positions of aligned lenses, and how these lenses correspond to the switches activated, and the sum total value produced by the electronic circuitry.

| **Aligned lenses and corresponding lens strength (dioptres)** | **Switches activated by presence of magnets M** | **Sum total value of activated switches** |
|---|---|---|
| A = +1.0 | SA | 1 |
| B = +1.25 | SB | 2 |
| C = +1.5 | SA, SB | 3 |
| D = +2.0 | SC | 4 |
| E = +2.5 | SA, SC | 5 |
| F = +3.0 | SB, SC | 6 |
| G = +3.5 | SA, SB, SC | 7 |

Hence, in this way, the position of the dials 28A, 28B is detected by the electronic circuitry, using a binary code. As each sum total value produced by a circuit corresponds to the alignment of a respective pair of lenses, the circuit effectively "knows" which lenses have been aligned by a user.

Hence, the electronic circuitry detects the position of the dials 28A, 28B by sensing which switches SA-SC are activated, and sums the values corresponding to each activated switch. The electronic circuitry is designed, on manufacture, to display the value "1.0" on the display 54 when the sum total value is 1, and to display "1.25" when the sum total value is 2, and so on. The numbers displayed on the display 54 in response to the summed values 1-7 can be altered if the lenses are replaced by others of different strengths, by making changes to the diodes within the electronic circuitry.

The electronic circuitry displays the lens strength only when the dials 28A, 28B are in one of the seven alignment configurations, by only activating the display 54 when the switch SD detects the contact presence of a raised portion 40 of a cog tooth 36. This is achieved by connecting the switch SD via a cable to the electronic circuitry, and by configuring the circuitry to only switch the display characters to "ON" when the switch SD is in contact with a raised area 40.

Because the position of activation of the switch SD corresponds to the position of engagement of the ball catch 46 with one of the grooves 44, the correct alignment configuration is assured.

Hence, the lens strength is only displayed when the selected lenses is in their most ideal position for making the sight test.

If the dials are in an intermediate position between two alignment configurations, no information (or an error message) is displayed, and the user becomes aware the dials are misaligned.

In addition to the above condition of being in an alignment configuration, the electronic circuitry only displays the lens strength when the battery voltage measuring circuit detects a voltage of 9V or higher, as will now be explained. The battery voltage measuring circuit determines when the voltage supplied to the electronic circuitry is too low for the light emitting diodes 20 to operate with the correct light level (set at 8.9V DC). When voltages below 8.9V and above 6V are sensed, the display will be driven to show a "low battery" signal, and blank all other parts of the display. The "low battery" signal will stay illuminated, even in the intermediate positions between alignment configurations, unless the electronic circuitry is in the stand-by mode discussed below. During the period when the "low battery" signal is illuminated, the two light emitting diodes 20 will be switched off.

When the voltage level is restored above +9V DC, the electronic circuitry will resume its normal operation.

The electronic circuitry has a powered up mode, in which the system is active, and a stand-by mode, in which the system conserves energy. This helps to prolong the life of the power supply.

The powered up mode is activated by movement of the dials 28A, 28B. That is, any change in signal from one or more of the switches SA, SB, SC, is detected by the electronic circuitry, causing activation of the powered up mode.

The electronic circuitry includes a timer that is adapted to activate the stand-by mode if the dials 28A, 28B have been stationary for a certain period of time, e.g. 60 to 90 seconds. In the stand-by mode, the electronic circuitry takes a current of around 150mA.

The electronic circuitry prevents power being supplied to the light emitting diodes 20 when the system is in the stand-by mode. Hence, energy is conserved during the periods when the system is not in use.

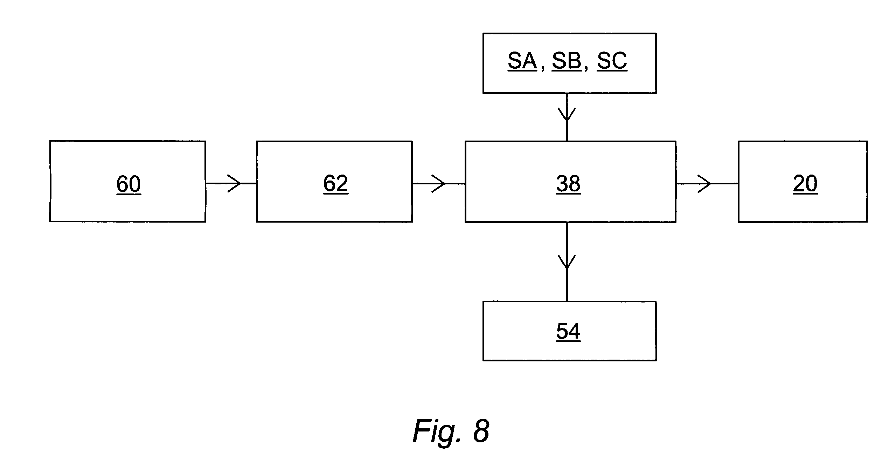

Fig 8 shows a flow chart of inputs and outputs to the circuit board 38. One input is power from the battery 60, which is transmitted via a power regulator 62. The power regulator 62 regulates the voltage to +5V. During normal operation it is estimated that the current consumption of the control and display functions will be in the region of 3.5mA. The electronic circuitry will operate down to a battery voltage of +6V DC.

Another input is the signals from the switches SA, SB, SC, which are then summed and converted to a lens strength, as described above. One output goes to the display 54, if the dials 28A, 28B are detected to be in one of the alignment configurations (dependent on the signals from SA, SB, SC) and if sufficient voltage is available from the battery 60. A further output goes to the light emitting diodes 20, if the electronic circuitry is in the powered up mode, and if a threshold voltage is available from the battery 60.

In use, the user rotates the dial 28B (which automatically also rotates the dial 28A) to select a pair of lenses for his sight test. The electronic circuitry detects changes in the signals from the microswitches SA, SB, SC and changes from the stand-by mode into the powered up mode. This causes the light emitting diodes 20 to light up to illuminate the reading chart 22. When the user rotates a pair of lenses into their alignment configuration, the electronic circuitry detects signals from the microswitches SA, SB, SC, sums these signals, and converts the summed total value into a lens strength, which is displayed on the display 54.

The user knows that the lenses are in the correct position when he feels resistance against turning the dial from the ball catch 46 engaging with the grooves 44. The user also knows that the lenses are in the correct position when a lens strength is displayed on the display 54.

The user then looks through the selected lenses. Light from the light emitting diodes 20 travels through the diffusers 34 and illuminates the reading chart 22. Light rays travel upwards from the reading chart 22, reflect through 90 degrees at the mirror 18, travel through the back plate 26, through the selected lens L mounted in the dial 28 and into the user's eye.

The user now attempts to read the reading on the reading chart 22, and makes an assessment about how suitable the selected lenses are. The user then typically rotates the dial 28B further, until another pair of lenses are in their alignment configuration, and repeats the sight test. Thus, the user can quickly and simply compare the seven different lenses to find the most suitable ones for his sight.

If it is desired to trial other lenses, the housing 12 can be opened, the lenses A-G removed, replacement lenses inserted, and replaced between the securing plates 50 and the system 10 reassembled.

If it is desired to trial lenses at other distances, the location of the reading chart 22 in the housing 12 can be adjusted, to alter the length of the visual path P.

Using a housing ensures that a reading chart can be located in the housing at a particular distance from the lens. Therefore, the distance between the lens and the reading chart (the visual path) can be controlled. This ensures that users do not inadvertently test different lenses at different distances from the reading chart, thereby arriving at a potentially erroneous result. Locating a light source within the housing means that the level of light under which sight tests are conducted can also be controlled, which helps provide consistent results, wherever the test is conducted.

The locating means (ball catch 46) effectively defines the discrete positions. The locating means can typically be arranged such that it locates the selected lens in its most ideal position, e.g. when the centre of the lens is exactly aligned with the visual path P to the reading chart 22.

This can ensure that the user will always be looking through the exact centre of the lens; hence, more accurate test results can be obtained.

The user only being given information on the lens strength when the selected lens is in its most ideal position for making the sight test, helps to alert the user if the dials are not correctly aligned, and therefore also helps to provide more accurate test results.

Modifications and improvements may be incorporated without departing from the scope of the invention. For example, the back plate 26 may be transparent, e.g. made of clear plastic, instead of being opaque with eye holes 26E.

The support mechanism does not necessarily comprise one or more rotary dials. Alternatively, any other means of presenting a lens to a user can be used. For example, the support mechanism could comprise an elongate conveyor or a continuous track conveyor.

More or fewer than seven lenses could be provided on each dial. More of fewer indicator elements may be used accordingly.

The locating means is not necessarily a ball catch.

Further alternatively, the switch SD could instead also take on the function of the locating means of holding the dials in one of the seven alignment configurations, against accidental rotation. For example, of the switch SD and the raised portions 40 were mutually attractive via magnetism, the switch SD would tend to hold the switch SD in contact therewith, against accidental movement of the dials 28A, 28B. Hence, it is clear that having a separate locating means is not an essential element of the invention, and any locating means could be used. Other embodiments do not have any locating means, and the dials are simply held in the alignment configuration by hand.

In a further alternative embodiment, the function of the switch SD can be incorporated within the ball catch 46 and groove arrangement 44. For example, contact switches could be located in the grooves 44, which are activated upon contact with the ball catch 46. In this way, the display 54 could be arranged to activate only when the ball catch 46 is engaged with a groove 44. In such an embodiment, the switch SD, the raised areas 40 and the grooves 42 would be redundent.

In an alternative embodiment, the electronic circuitry comprises a Central Processing Unit (CPU). The CPU may be programmed to take in information relating to the positions of the dials, process this information and calculate a required response based on this. The CPU may also include more optional statements for display. For example "low battery", "error", or a welcome greeting.

Optionally, the light emitting diodes 20 and reading chart 22 are mounted in an internal carriage that is moveable within the housing 12, to enable easy adjustment of the position of the reading chart 22, and hence the length of the visual path P.

In alternative embodiments, the ball catch may be mounted on the moveable part of the support mechanism (e.g. the dial) and the grooves are located on the fixed part of the support member (e.g. the back plate).

It is not essential to set the current for the light emitting diodes 20 at 22mA. The minimum luminescence level to adequately illuminate the reading chart 22 will vary according to the type of light emitting diodes used.

In an alternative embodiment, the magnets M could be replaced with holes or raised segments, the presence or absence of which can be detected by optical sensors. Optionally, two, three or more optical sensors could be provided on the back plate 26, along lines corresponding to one or more of the alignment axes X1 A-X7A, X1 B-X7B.

Some embodiments may only present one lens at a time to a user, instead of two.

Some embodiments may have more or fewer indicator elements (e.g. Magnets) and a greater or larger selection of lenses. If necessary, the binary code system can be extended accordingly, e.g. an additional set of Magnets and a further switch could be provided for the dial 28B, this further switch producing a value of 8 in response to detection of an adjacent magnet M.

In alternative embodiments, the inner and outer magnets M on dial 28A need not be spaced along the same radial axis of the dial.

The housing 12 is not necessarily L-shaped, and could have other shapes.

## Claims

1. A lens selection system, comprising:
a housing;
at least one lens arranged to allow a view into the housing by a user;
a support mechanism on which the least one lens is mounted, the support mechanism having a part that is fixed relative to the housing; and
a light source located within the housing.

2. A lens selection system as claimed in claim 1, wherein the light source comprises a light emitting diode.

3. A lens selection system as claimed in claim 2, including a light control means for controlling the level of light inside the housing.

4. A lens selection system as claimed in claim 3, wherein the light control means comprises electronic circuitry and a power regulator;
wherein the electronic circuitry controls the flow of current to the light emitting diode, and
wherein the power regulator is connected to the light emitting diode, to provide a stable power supply to drive the light emitting diode.

5. A lens selection system as claimed in claim 4, wherein the electronic circuitry is provided with a power supply and the electronic circuitry also includes means to ensure the light source will be activated only when a threshold voltage is provided by the power supply.

6. A lens selection system as claimed in any of claims 3 to 5, wherein the system has a powered up mode and a stand-by mode and wherein the light control means prevents power being supplied to the light source when the system is in the stand-by mode.

7. A lens selection system as claimed in any preceding claim, wherein a plurality of lenses are mounted on the support mechanism, and wherein the support mechanism has a part that is moveable with respect to the housing to present a selected lens to the user.

8. A lens selection system as claimed in claim 7, wherein the moveable part of the support mechanism is moveable into a number of discrete positions.

9. A lens selection system as claimed in claim 8, wherein the system includes a locating means that resists movement of the moveable part of the support mechanism away from the discrete positions.

10. A lens selection system as claimed in claim 9, wherein the locating means comprises a catch and a series of grooves, the catch being selectively engageable with the grooves.

11. A lens selection system as claimed in any of claims 8 to 10, including electronic circuitry that includes a detector for detecting the position of the moveable part of the support mechanism.

12. A lens selection system as claimed in claim 11, including electronic circuitry which comprises:
means to convert the detected position of the support mechanism into a corresponding lens strength; and
a display for presenting information relating to the lens strength to the user.

13. A lens selection system as claimed in claim 12, wherein the electronic circuitry is adapted to display information relating to the lens strength only when the support mechanism is in one of the discrete positions.

14. A lens selection system as claimed in any preceding claim, wherein a reading chart is located inside the housing and wherein at least one mirror is located in a visual path between the lens and the reading chart.

15. A lens selection system as claimed in claim 14, wherein the length of the visual path between the lens and the reading chart is adjustable.
